# EUROPÄISCHE PATENTSCHRIFT

(11) **EP 0 677 044 B1**
(45) Veröffentlichungstag und Bekanntmachung des Hinweises auf die Patenterteilung: **28.10.1998**
(21) Anmeldenummer: 94903874.9
(22) Anmeldetag: 24.12.1993
(51) Int. Cl.: C12P 1/00, C12N 9/96, A61K 31/505, A61K 31/14, C07C 257/10, C07D 239/06, C12N 1/04, A61K 7/00

(54) **VERFAHREN ZUR IN VIVO GEWINNUNG VON INHALTSSTOFFEN AUS ZELLEN**
(IN VIVO) METHOD OF OBTAINING CELL COMPONENTS
PROCEDE PERMETTANT D'OBTENIR (IN VIVO) DES CONSTITUANTS DE CELLULES

(30) Priorität: 31.12.1992 DE 4244580
(43) Veröffentlichungstag der Anmeldung: 18.10.1995
(60) Teilanmeldung: 98113132.9
(73) Patentinhaber: bitop Gesellschaft für biotechnische Optimierung mbH, 58455 Witten (DE)
(72) Erfinder: Galinski, Erwin, A., 53127 Bonn (DE); Truper, Hans, G., 53177 Bonn (DE); Sauer, Thomas, 53121 Bonn (DE)
(74) Vertreter: Meyers, Hans-Wilhelm, Dr.Dipl.-Chem.
(86) Internationale Anmeldenummer: EP9303687
(87) Internationale Veröffentlichungsnummer: WO9415923

(56) Entgegenhaltungen:
- EP-A- 0 200 032
- WO-A-92/04054
- DE-A- 3 820 595
- FR-A- 2 635 113
- US-A- 4 286 660
- US-A- 4 720 458
- CHEMICAL ABSTRACTS, vol. 104, no. 11, 17. März 1986, Columbus, Ohio, US; abstract no. 85323w, REED R. ET AL. 'Osmotic shock-induced release of low molecular weight metabolites from free-living and immobilized cyanobacteria.' Seite 375 ; & ENZYME MICROB.TECHNOL., Bd.8, Nr.2, 1986 Seiten 101 - 104
- CHEMICAL ABSTRACTS, vol. 117, no. 17, 26. Oktober 1992, Columbus, Ohio, US; abstract no. 167591q, SEVERIN JOERG ET AL. 'The predominant role of recently discovered tetrahydropyrimidines for the osmoadaptation of halophilic eubacteria.' Seite 461 ; & J.GEN.MICROBIOL., Bd.133, Nr.8, 1992 Seiten 1629 - 1638
- CHEMICAL ABSTRACTS, vol. 115, no. 3, 22. Juli 1991, Columbus, Ohio, US; abstract no. 29378h, Seite 784 ; & JP,A,03 031 265 (TAKEDA) 12. Februar 1991
- CHEMICAL ABSTRACTS, vol. 104, no. 11, 17. März 1986, Columbus, Ohio, US; abstract no. 83993r, SCHUH, W. ET AL. 'The crystal structure of ectoine , a novel amino acid of potential osmoregulatory function.' Seite 259 ; & Z.NATURFORSCH.,C:BIOSCI., Bd.40C, Nr.11, 1985 Seiten 780 - 784

## Beschreibung

Gegenstand der vorliegenden Erfindung ist ein Verfahren zur in vivo Gewinnung von Inhaltsstoffen aus Zellen.

Die gebräuchlichen biotechnologischen Verfahren zur Gewinnung niedermolekularer Metaboliten im technischen Maßstab nutzen fehlregulierte/überproduzierende oder "leaky" Mutanten, die das entsprechende Produkt ins Medium ausscheiden. Eine erfolgreiche Mutagenisierung und Mutantenselektion setzt jedoch voraus, daß ein geeigneter Produkt-Schnelltest zur Verfügung steht, oder daß mit spezifischen Antimetaboliten ein Selektionsdruck ausgeübt werden kann. Diese Voraussetzungen sind im allgemeinen jedoch für viele Produkte nicht gegeben. Mithin muß in zunächst aufwendigen Verfahren dafür Sorge getragen werden, daß spezielle Mutanten gezüchtet werden, die diese Bedingungen für die entsprechende Produkte erfüllen. Des weiteren ist nachteilig, daß das Produkt bei sogenannten Dauerausscheidern aus einem mehr oder weniger komplexen Kulturmedium isoliert werden muß.

CA Vol. 104, no. 11, 1986, abstr. no. 85323w und Reed et al., Enzyme Microb. Technol. 8 (1986), S.101-104 beschreibt ein Verfahren zur Freisetzung von Carbohydraten mit geringem Molekulargewicht und freien Aminosäuren aus den einzelligen Cyanobakterien Synechocystis PCC6714 und Synechococcus PCC6311.

Das der Erfindung zugrunde liegende Problem ist die Bereitstellung eines prozeßtechnisch steuerbaren Verfahrens mit dem aus Zellen in kontinuierlicher Weise Zellinhaltsstoffe isolierbar werden, wobei das Verfahren in vorteilhafter Weise zyklisch gestaltet werden kann und somit wirtschaftlich ist. Das erfindungsgemäße Verfahren soll eine in-vivo-Isolierung der entsprechenden Produkte ermöglichen, so daß die Zellen nicht zerstört, abgetrennt und durch neue Produzenten ersetzt werden müssen.

Überraschenderweise wird dieses technische Problem gelöst durch ein Verfahren zur in-vivo-Gewinnung von Inhaltsstoffen mit den Merkmalen des Anspruchs 1. Die Ansprüche 2 bis 15 betreffen bevorzugte Ausführungsformen des erfindungsgemäßen Verfahrens.

Das erfindungsgemäße Verfahren zur in vivo Gewinnung von Inhaltsstoffen aus Zellen geht davon aus, daß vorkultivierte Zellen, die in einem bestimmten ersten Zustand gewachsen sind, in einen zweiten Zustand überführt werden. Durch diesen Schock werden die Zellen gezwungen, durch Abgabe von Zellinhaltsstoffen in die Umgebung zu reagieren, um eine Anpassung der Zellen an die Bedingungen, die im zweiten Zustand herrschen, zu erreichen. Dabei müssen die vorgenannten Verfahrensschritte mindestens zweimal durchlaufen werden (Wechselschock).

Die Veränderung in der Umgebung der Zellen im ersten Zustand kann beispielsweise durch Veränderung des Druckes, der Temperatur, der Konzentration von Stoffen, dem pH-Wert oder Kombinationen dieser Maßnahmen erzielt werden.

So ist es beispielsweise möglich, einen Schock durch Änderungen der osmotischen Bedingungen im Kulturmedium auszulösen.

Das erfindungsgemäße Verfahren kann vorzugsweise mit weiteren Maßnahmen kombiniert werden, wie solchen, die eine Erhöhung der Permeabilität von Zellmembranen hervorrufen, wie beispielsweise Elektroporationsverfahren. Die Permeabilität der Zellmembranen kann auch durch permeabilisierende Substanzen verändert werden. Dazu gehören insbesondere porenbildende und/oder diffusionsfördernde Peptid- und Depsipeptidantibiotika, z.B. Valinomycin, Polymyxin, Gramicidin, Nisin sowie Detergenzien wie z.B. Natriumdodecylsulfat, Triton X, Cetyltrimethylammoniumbromid. Auch Wachstum der Zellen in Mangelmedien kann zu einer Erhöhung der Zellpermeabilität führen. Dies geschieht insbesondere dann, wenn den Zellen bestimmte Nährstoffe, wie Biotin oder Mangan, vorenthalten werden. Auch durch Verwendung von Mutanten, deren Lipidstoffwechsel gestört ist, kann die Zellmembran der betreffenden Zellen durchlässiger sein.

Aufgrund der Tatsache, daß die Zellen nicht zerstört werden, sondern lediglich durch Leckagen, die in Form von spezifischen oder unspezifischen Poren oder aktiven Transportsystemen vorliegen können, Zellinhaltsstoffe ins Medium abgeben, kann durch Regeneration des ersten Zustandes und erneuten Wechsel in den zweiten Zustand das erfindungsgemäße Verfahren kontinuierlich gestaltet werden. Dies erfolgt erfindungsgemäß in Form von sogenannten Wechselschocks in zyklischer Prozeßführung. Vorzugsweise werden zwischen den Wechselschocks die Zellen solange bei konstanten Bedingungen gehalten, bis zwischen Zellen und Umgebung quasi-stationäre Bedingungen vorliegen.

Als Zellen, die dem erfindungsgemäßen Verfahren unterworfen werden können, kommen Mikroorganismen, insbesondere Bakterien, Pilze, Hefen, Algen, Rotalgen oder Zellen höherer Organismen, tierische oder pflanzliche Gewebe, oder Organe in Betracht.

In bestimmten Konstellationen kann es vorteilhaft sein, als Zellen genetisch manipulierte Zellen einzusetzen, die bestimmte Stoffe synthetisieren und diese in Folge des Schocks in die Umgebung sezernieren.

Nach dem erfindungsgemäßen Verfahren arbeitet man in einer bevorzugten Ausführungsform, wie im folgenden beschrieben. Beispielsweise produzieren halotolerante Eubakterien in Anpassung an hohe Salinität niedermolekulare Naturstoffe (Osmotika), die native Enzyme in einem Milieu geringer Wasseraktivität stabilisieren. Sie können als Schutzstoffe in der Enzymtechnologie eingesetzt werden, und zwar als Hitze-, Gefrier- und Trocknungsschutzsubstanzen. Viele dieser "Solute", wie beispielsweise Verbindungen aus der Klasse der Tetrahydropyrimidincarbonsäuren, sind biotechnologisch in Anwendung des erfindungsgemäßen Verfahrens leicht zugänglich. Das erfindungsgemäße Verfahren nutzt die Fähigkeit der halotoleranten Organismen, bei einem Verdünnungsstreß rasch große Mengen organischer Osmotika zu exportieren, um osmotisch bedingtes Platzen der Zellen zu vermeiden. Vorteilhaft am erfindungsgemäßen Verfahren ist die Tatsache, daß die Ausscheidung der zytoplasmatisch akkumulierten Substanzen auch bei Wildstämmen induzierbar ist, und daß das Produkt in relativ reiner Form in einem verdünnten wäßrigen Medium vorliegt. Die problematische Reinigung niedermolekularer organischer Verbindungen wird dadurch wesentlich vereinfacht. Da die Produktion beispielsweise der Solute vorrangig und unabhängig vom Zellwachstum erfolgt, läßt sich die in-vivo-Extraktion, in Verkörperung des erfindungsgemäßen Verfahrens, beliebig oft wiederholen und kann in einem kontinuierlichen Verfahren der Biokonversion mit Biomasserecycling weitergeführt werden.

In einer bevorzugten Ausführungsform des erfindungsgemäßen Verfahrens werden Mikroorganismen, wie Proteobakterien, insbesondere Halomonadaceae, oder Firmicutes, z.B. Micrococci, eingesetzt.

Durch stufenweises Verdünnen einer dichten Zellkultur, Analyse der ausgeschiedenen Zellinhaltsstoffe und abschließender Lebendzellzahlbestimmung kann ein geeigneter Organismus identifiziert werden, der auch extremen Verdünnungstreß toleriert.

Die Zellen werden vorzugsweise von einer Umgebung mit einer Osmolarität von bis zu 30 Osmol in eine Umgebung möglichst geringer Osmolarität überführt. Die Osmolarität kann auch zwischen 5 und 1 Osmol geändert werden.

Ein bevorzugter Organismus sollte auch ohne komplexe Nährstoffe schnell wachsen, das gewünschte Produkt gegebenenfalls aufgrund genetischer Manipulation in hoher Konzentration im Zytoplasma anreichern können (bis zu 2 M) und Wechselschocks, vorzugsweise zwischen 20 %iger Natriumchloridlösung und reinem Wasser, unbeschadet überstehen. Durch die Inkubation in niederosmolarem Nährmedium (idealerweise Wasser) werden die Zellen zur Abgabe ihrer Osmotika gezwungen. Das von den Zellen getrennte niederosmolare Medium enthält die Osmotika (Produktlösung). Die zyklische Prozeßführung ermöglicht eine wirtschaftlichere Verfahrensführung, da die einmal kultivierten Zellen mehrfach zur Produktion der Osmotika eingesetzt werden können. Ein Aufschluß der Zellen entfällt ebenso wie die Abtrennung von Produkt und Zelltrümmern.

Die zum Austausch der Nährmedien erforderliche Abtrennung von Zellen vom Außenmedium kann auf verschiedene Weise erfolgen. Darunter sind Methoden zu nennen wie Zentrifugation, Filtration, Ausflockung oder Immobilisierung der Zellen. Die Veränderung der Osmolarität kann aber auch durch eine veränderliche Löslichkeit der Osmotika im Außenmedium, z.B. durch Temperatur, durch deren Polymerisationsgrad oder Komplexierung erreicht werden. Entsprechend der Wahl des Trennungsverfahrens weist der Anlagenaufbau zur Kultivierung der Zellen bestimmte Unterschiede auf. Vorteilhaft ist eine hohe Beladung der Anlage mit Zellen, da auf diese Weise die Ausbeute gesteigert werden kann. Die hohe Zelldichte kann z.B. durch eine Hochzelldichtefermentation oder ein Immobilisierungsverfahren, das zu einem hohen Zellanteil im Immobilisat führt, erreicht werden.

Verbindungen mit dem Strukturelement Formel I, sind beispielsweise Naturstoffe, die aus entsprechenden Kulturen isoliert werden können. Verbindungen, die das Strukturelement

R¹-N=C(R²)-NHR³ I

enthalten, können in protonierter Form mit den prototropen Strukturen (II) und (III) wiedergegeben werden,

R¹⁺NH=C(R²)-NHR³ II <-> R¹-N=C(R²)-⁺NH₂R³ III,

Dabei sind R¹ und/oder R³ Alkyl-, Cycloalkyl oder mit Heteroatomen substituierte oder funktionalisierte Derivate, wobei die Derivatisierung mit Hydroxy-, Sulfonsäure-, Carbonsäurederivate, wie Amide, Ester usw., Carbonyl, Ether, Alkoxy- und andere Hydroxylgruppenderivate erfolgt ist und wobei R¹ und R³ zusammen mit dem Strukturelement gemäß Formel I einen Ring bilden können
sowie R² Wasserstoff ist oder eine der für R¹, R³ genannte Bedeutungen besitzt.

Die Verbindungen können zur Stabilisierung von Enzymen und anderen Biomolekülen in wäßrigen Lösungen und organischen Lösungsmittel sowie zur Stabilisierung gegen denaturierende Bedingungen verwendet werden.

Als Biomoleküle kommen neben Enzymen beispielsweise Proteine oder weitere Biopolymere in Frage. Als denaturierende Reagenzien sind Salze, extreme pH-Werte, Tenside, Harnstoff, Guanidiniumchlorid und andere Verbindungen zu nennen. Die Verwendung der genannten Stoffe mit dem Strukturelement I erlaubt die Erhaltung der komplexen Struktur der Biomoleküle selbst unter den genannten Bedingungen.

Vorzugsweise werden Verbindungen eingesetzt, die das Strukturelement I in einem 6- bis 8-gliedrigen System enthält. Als besonders bevorzugte Substanzen haben sich Substanzen aus der Klasse der Ectoine, insbesondere Ectoin und Hydroxyectoin bewährt.

Desweiteren kommen Verbindungen in Betracht, die an den funktionellen Gruppen der genannten Ectoinverbindungen derivatisiert sind. Im Falle des Hydroxyectoins können beispielsweise aus der Hydroxylgruppe Ether oder Ester gebildet werden. Aus der Carboxylgruppe können Amide oder Ester gebildet werden. Die Carboxylatgruppe kann ersetzt werden durch eine Carbonylgruppe oder eine Sulfonsäuregruppe bzw. deren Ester und Amide.

Als extrahierbare Naturstoffe aus Bakterienkulturen kommen insbesondere Osmotika der folgenden Gruppen in Betracht: Ectoine, insbesondere Ectoin und Hydroxyectoin, die als hygroskopische Verbindungen eingesetzt werden können; Aminosäuren und ihre Derivate, insbesondere N-Acetylierte Diaminosäuren, wie N-Acetyl-Lysin und N-Acetyl-Ornithin, N-modifizierte Dicarboxamide; Zucker und ihre Derivate, beispielsweise Trehalose (Trockenstabilisator), Polyole, Betaine, Peptide und Proteine.

Insbesondere wird von Halomonas elongata und Marinococcus halophilus das optisch aktive Hydroxyectoin der nachstehenden Formel gebildet. 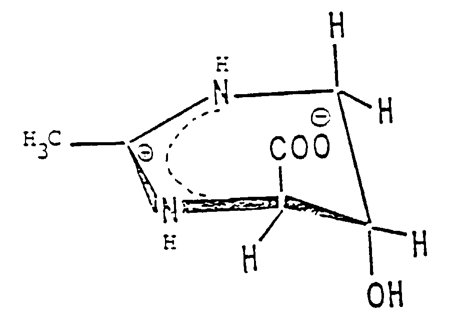 S,S-α-Amino-β-hydroxyectoin

Diese Substanz ist beispielsweise neben anderen Ectoin-Derivaten zur Kryoprotektion von biologischen Wirkstoffen, Zellen, Blutkonserven usw. einsetzbar und kann in Medizintechnik, Kosmetik und pharmazeutische Industrie Verwendung finden.

Ectoin und Ectoin-Derivate wie Hydroxyectoin können auch in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutisch wirksame Peptide und Proteine z.B. t-PA können mit Ectoin oder seinen Derivaten geschützt werden.

Ein typischer Verfahrensverlauf am Beispiel der Kultivierung von Halomonas elongata wird im folgenden beschrieben.

Die Vorkultur von Halomonas elongata wird im Bioreaktor im Batchverfahren angezüchtet. Die Batchfermentation wird solange durchgeführt bis ein Großteil der Kohlenstoffquelle, in Form von beispielsweise Glucose, verbraucht ist.

Viele wichtige Nährlösungsbestandteile, wie z.B. Glucose oder Methanol, lassen sich nur in begrenzter Menge einsetzen, da sie in zu großer Konzentration wiederum das Wachstum der Mikroorganismen hemmen. Um dennoch hohe Zelldichten zu erreichen, kann man solche das Wachstum limitierende Stoffe während der gerammten Fermentationsdauer kontinuierlich zufüttern ("Fed-Batch-Kultur"). Erfolgt das Zufüttern mit exponentiell ansteigender Fütterungsrate, so wird das exponentielle Wachstum der Mikroorganismen optimal unterstützt und es wird eine maximale Raum-Zeit-Ausbeute erreicht. Wählt man die Parameter richtig, so kann zu jedem Zeitpunkt des exponentiellen Wachstums die entsprechende Substratmenge zugefüttert werden. Werden die Parameter nicht optimal abgeglichen, kann im Extremfall Substratakkumulationen im Reaktor auftreten und damit das Wachstum der Mikroorganismen gehemmt werden, bis hin zum Absterben der Mikroorganismen.

Die optimale Fütterungsstrategie kann beispielsweise nach dem Algorithmus von Keller und Dunn erfolgen (Keller R. and Dunn I.J. Fed-batch microbial culture: models, errors and applications. J. appl. Chem. Biotechnol., 28: 508 bis 514, (1978)).

An die Hochzelldichtefermentation schließt sich der Verfahrensschritt der Konzentrierung der Zellsuspension an. Um anschließend die Solute aus den Zellen zu extrahieren, ist es empfehlenswert, zunächst den Großteil des Außenmediums zu entfernen. Diese Konzentrierung der Zellsuspension kann insbesondere durch Querstromfiltration durchgeführt werden. Dazu wird die Zellsuspension mittels einer Fördereinrichtung, wie einer Pumpe, durch eine Filtriereinrichtung, beispielsweise eine Filterkassette, gefördert.

Querstromfiltrationen finden seit einigen Jahren vielfältige Anwendung, z.B.:
- in der Enzymgewinnung aus Zellhomogenisaten
- zur Zellernte sowohl von Bakterien- als auch von Tierzellkulturen
- in Fermentationen mit Zellrückhaltung zur Steigerung der Zelldichte (Katalysatordichte) und bei gleichzeitiger Abführung des (inhibierenden) Produkts im Filtrat.

Nach der Konzentrierung der Zellen werden diese einem hypoosmotischen Schock ausgesetzt. Der Verdünnungstress wird beispielsweise durch Zugabe von sterilem Aqua destillata bis zum Ausgangsvolumen der Kulturflüssigkeit herbeigeführt. Vorzugsweise wird das Wasser innerhalb eines Zeitraumes von weniger als 30 Minuten zugegeben.

Daraufhin erfolgt vorzugsweise eine zweite Konzentrierung der Zellsuspension, die analog zur ersten Filtration durchgeführt werden kann. Das nunmehr abfiltrierte Außenmedium enthält das Produkt und wird aufgefangen. Die Produktlösung kann dann nach an sich bekannten Verfahren aufgearbeitet werden.

Daran anschließend wird vorzugsweise bei kontinuierlicher Verfahrensführung ein hyperosmotischer Schock ausgeübt, indem eine hochkonzentrierte Nährstofflösung mit hohem Salzgehalt wiederum bis zum Ausgangsvolumen zugegeben wird. Es kann sich empfehlen, die Zugabe in einem längeren Zeitintervall durchzuführen als bei Ausführung des hypoosmotischen Schocks.

Es kann vorteilhaft sein, nach dem hyperosmotischen Schock die Zellen in einer Regenerationsphase zu halten, um eine vollständige Synthese der kompatiblen Solute im Zellinnern zu erleichtern. Danach ist die Zellkulturlösung zu einem weiteren Zyklus bereit, der aus den Schritten der Konzentrierung der Zellen, dem hypoosmotischen Schock, Filtration der Produktlösung, hyperosmotischen Schock und der Regenerationsphase besteht.

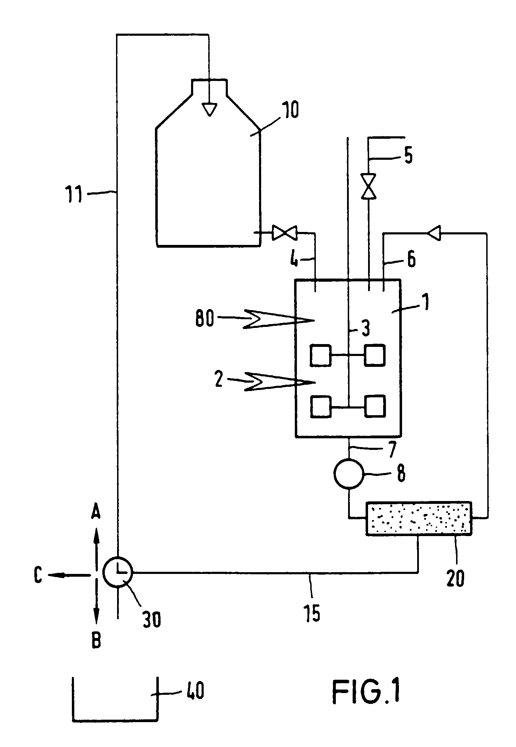

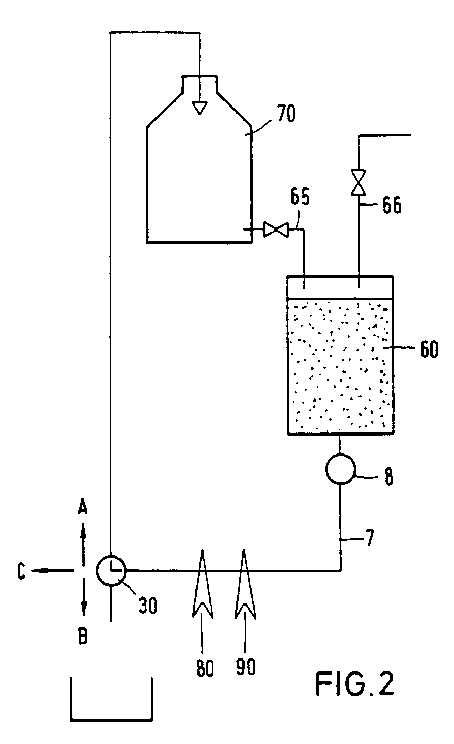

Die Figuren 1 und 2 zeigen schematische Darstellungen der Prozeßführung im Filtrationsmodus (Figur 1) und im Festkörperverfahren (Figur 2).

Figur 1 beschreibt eine Anordnung bestehend aus einem Fermenter 1, in dem die Bakterienkultur gezüchtet wird. Der Fermenter ist mit einem Trübungsmeßsystem 2, einem Leitfähigkeitsmeßsystem 80, einem Rührer 3 und den Zu- und Ableitungen 4, 5, 6 und 7 versehen. Das Vorratsgefäß 10 steht über die Zuleitung 4 mit dem Fermenter in Verbindung und weist vorzugsweise darüberhinaus eine Verbindung 11 zu der Querstromfiltrationseinheit 20 über ein Mehr-Wege-, insbesondere 3-Wege-Ventil, auf.

Über die Pumpe 8 kann das Medium durch die Querstromfiltrationseinheit 20 abgenommen und der Reaktorinhalt konzentriert werden. Über die Leitung 15 kann dann das abgezogene Medium über das 3-Wege-Ventil 30 in den Vorratsbehälter 10 überführt (Stellung A) oder aufgefangen und gegebenenfalls nach Aufarbeitung (Reinigung) wiederverwendet werden (Stellung C). Über die Zuleitung 5 wird danach der hypoosmotische Schock ausgelöst, indem Wasser zugeleitet wird bis das ursprüngliche Volumen im Reaktionsgefäß 1 wieder hergestellt ist. Danach kann erneut über die Ableitung 7 und die Querstromsfiltrationseinheit 20, das nunmehr osmotisch verdünnte Kulturmedium entfernt werden und dann über eine alternative Schaltung des 3-Wege-Ventils 30 (Stellung B) zur Produktseite in ein entsprechendes Auffanggefäß 40 überführt werden.

Die ursprünglichen osmotischen Verhältnisse können durch Zugabe des Mediums aus dem Vorratsbehälter 10 bzw. hochsalines Medium wiederhergestellt werden.

In einer anderen Ausführungsform, die in Figur 2 dargestellt ist, ist die Festkörper- oder Wirbelschichtkultur 60 mit den Zuleitungen 65, 66 ausgestattet. Die Kultur 60 besteht aus den entsprechenden immobilisierten Organismen. Die Zuleitung 65 ist mit einem Vorratsgefäß 70 verbunden, in dem die Nährlösung bevorratet werden kann. Über die Zuleitung 66 kann das Medium für den hypoosmotischen Schock zugeleitet werden.

Über die Pumpe 8 kann das Medium jeweils abgezogen werden und je nach Verfahrenschritt entweder über das 3-Wege-Ventil 30 in das Vorratsgefäß 70 zurückgeführt, aufgefangen und aufgearbeitet werden (Stellung C) oder in Stellung B des 3-Wege-Ventils die Produktlösung aufgefangen werden. Vorzugsweise sind in der Ableitung 7 Meßeinrichtungen wie Leitfähigkeitsmeßsysteme 80 oder Durchflußphotometer 90 zur Kontrolle der Salinität bzw. Produktmonitoring angeordnet.

Ebenfalls können weitere Meßsonden, -fühler angeordnet sein. Optional sind jeweils auch Steuereinheiten anschließbar.

### Versuchsbeispiel:

**1,5 l -Bioreaktor:** Biostat M (B. Braun, Melsungen) mit Meßaufsatz HE6. Im Bioreaktor wurden über eine große Zahl von Sonden Meßdaten erfaßt: pH-Sonde, pO₂-Sonde, OD-Sonde, Schaumsonde, Temperatursonde und Leitfähigkeitssonde. Die Meßdaten werden zum zweiten wesentlichen Bestandteil der Anlage, dem Fermenterleitsystem, weitergeleitet.
**Fermenterleitsystem:** (Münzer & Diehl, Overath), bestehend aus einem Batch-Controller (zur Datenerfassung und -digitalisierung sowie zur Steuerung und Regulation von Prozeßgrössen) und einem IBM-Industriecomputer AT 286 mit FAS-(Fermenter Application software)-Programm (zur Visualisierung und Speicherung der Daten sowie zur Programmierung des Batchcontrollers).
**Querstromfiltration:** Querstromfiltrationseinheit Minisette Sanitary Cell System mit Filtratonskassette (SS994C02, Porengröße 0,16 µm, Filterfläche 700 cm², FILTRON, Northborough, MA, U.S.A.)
   In diesem Verfahren dient die Querstromfiltration der Konzentrierung der Bakteriensuspension im Fermenter. Der Druck in der Querstromeinheit wird mit Hilfe von Manometern kontrolliert und durch Ventile reguliert.
**weitere Geräte:**
   - Trübungsmeßgerät AS 82 und Trübungsmeßsonde AF 44 S (IMA, Neu-Isenburg-Zeppelinheim) nach dem Prinzip der Streulichtmessung
   - Leitfähigkeitsmeßgerät LF 537 und Leitfähigkeitsmeßsonde Tetracon 96 (WTW, Weilheim)
   - Schlauchpumpe 2006 (VERDER, Düsseldorf)
   - Schlauchpumpe MV-CA 4 (ISMATEC, Glattbrugg, CH)
   - 2 Membranpumpen FE 211 (B. Braun, Melsungen).
**Lösungen:** Jede Fermentation wurde mit 200 ml Vorkultur und 1000 ml VVM15 gestartet (verändertes VREELAND-Medium VVM15, modifiziert: NaCl (145,8 g), KCl (1,0 g), MgSO₄•7 H₂O (6,5 g), NH₄Cl (3,0 g), K₂HPO₄ (0,5 g), CaCl₂•2 H₂O (0,01 g), FeSO₄•7 H₂O (0,01 g), Glucose-Monohydrat (11,0 g), TES (1,0 ml), Aqua dest. ad 1000 ml, pH 7,0 (mit 1 N HCl), als Vorkulturmedium mit 6 g/l TRIS und 1 N HCl auf pH 7,5 gepuffert, TES: Spurenelementlösung, Medium Nr. 141, DSM 1989).
   Zur Regulation des pH-Wertes während der Versuche wurden NaOH und HCl eingesetzt. Zur Durchführung der Hochzelldichtefermentation und der Wechselschockschritte wurden FBL-Medium (Glucose-Monohydrat (297,3 g), NH₄Cl (66,9 g), K₂HPO₄ (43,5 g), 1 N HCl (100 ml), Aqua dest. ad 500 ml), und KS30-Medium zur Regulation der Salinität (NaCl (300 g), KCl (2 g), MgSO₄•7 H₂O (13 g), CaCl₂ • 2 H₂O (0,02 g), FeSO₄•7 H₂O (0,02 g), TES (2 ml), Aqua dest. ad 1000 ml, pH 7) sowie Aqua dest. benötigt.
**Batch-Kultur**: Der erste Verfahrensschritt war die Anzucht von Halomonas elongata im Bioreaktor im Batchverfahren. Die Fermentationsbedingungen während der gerammten Fermentation waren: pH 7.5, 25°C, Belüftungsrate 0.16 l/min Luft, 15 % NaCl. Die Batchfermentation wurde solange durchgeführt, bis ein Großteil der Glucose verbraucht war.
   Die **Fütterungsstrategie** kann nach Keller & Dunn ermittelt werden.
   Für die auftretenden Parameter Startbiomassekonzentration (Xₒ), Startvolumen (Vₒ); Wachstumsrate, Ertragskoeffizient und Substratkonzentration müssen die geeigneten Werte gesucht werden. Die Startparameter Biomassekonzentration (Xₒ) und Startvolumen (Vₒ) wurden am Ende der Batchphase ermittelt. Der Ertragskoeffizient Y_{XS} beträgt beim Wachstum von Halomonas elongata in Glucose/Mineralsalz-Medium 0,5. Die Substratkonzentration Sᵢₙ in der FBL betrug 540 g/l; die Substratkonzentration S im Fermenter war am Ende der Batchphase nahe 0 und sollte durch die Fütterungsstrategie nicht groß werden. Deshalb wurde S für die Berechnung der Fütterungsrate vernachlässigt. Der letzte Startparameter der Fütterungsstrategie, die Wachstumsrate, wurde empirisch bestimmt.
   Die Fütterungsstrategie wurde in das FAS programmiert und die Fütterungsrate mit Hilfe eines ebenfalls programmierten Zeittaktes für jeden Zeitpunkt der Hochzelldichtefermentation berechnet. Über den Batchcontroller wurde die Fütterungsrate an eine Membranpumpe übergeben und die Fedbatchlösung mit der entsprechenden Flußrate in den Fermenter gepumpt. Es wurden Zelldichten bis zu 120 g/l Frischgewicht (entspricht 40 g/l Trockengewicht) erreicht.
**Konzentrierung der Zellsuspension:** Um anschließend die Solute aus den Zellen zu extrahieren, mußte ein Großteil des Außenmediums entfernt werden. Diese Konzentrierung der Zellsuspension wurde mit einer Querstromfiltrationsanlage durchgeführt. Dazu wurden die Zellsuspension mit einer Schlauchpumpe durch die Filterkassette gepumpt. Der Druck in der Filterkassette wurde so reguliert, daß er am Ausgang des Filtermoduls um etwa 0,5 bar geringer als im Eingang war. Er durfte jedoch 3 bar nicht übersteigen. Die Filtrationsdauer hing vom Zustand der Filterkassette ab und schwankte zwischen 30 min bei neuen Kassetten und 2 h bei älteren. Für die Dauer der Filtration wurde die Drehzahl des Rührers im Fermenter auf 300 UpM reduziert und die Zuluft vollständig abgestellt, um ein Eindringen von Luftblasen in den Filter zu verhindern. Das Volumen der Zellsuspension wurde durch die Filtration auf 1/5 des Ausgangsvolumens reduziert (Dies entspricht einer Zelldichte von bis zu 600 g/l Frischgewicht).
**Hypoosmotischer Schock:** Der Verdünnungsstreß wurde durch die Zugabe von sterilem Aqua dest. bis zum Ausgangsvolumen herbeigeführt. Das Wasser wurde während eines Zeitraums von 10 bis 30 min zugegeben.
**Filtration der Produktlösung:** Die zweite Konzentrierung der Zellsuspension wurde durchgeführt, um die Produktlösung von den Zellen abzutrennen. Sie verlief analog zu der ersten Filtration, mit dem Unterschied, daß das abfiltrierte Außenmedium nun das Produkt enthielt. Es konnte eine Konzentration von bis zu 3,7 g Ectoin/1 in der Produktlösung erzielt werden.
**Hyperosmotischer Schock:** Der Salzstreß wurde durch die Zugabe von VVM18 (Glucose/Mineralsalzmedium mit 18 % (w/v) Salz) bis zum Ausgangsvolumen ausgelöst. Die Zugabe erfolgte über einen Zeitraum von 2 h mit konstanter Flußrate, woraus sich ein zeitlicher Gradient in der Salzkonzentration des Mediums ergibt.
**Regenerationsphase:** Im Anschluß an den hyperosmotischen Schock folgte eine Regenerationsphase von etwa 24 h zur vollständigen Synthese der kompatiblen Solute. Während dieser Zeit wurde mit einer konstanten Flußrate Fedbatchlösung zugefüttert, so daß 1,3 g Glucose pro Stunde zugefüttert wurden.
**Wechselschockzyklen**: Nach der Regenerationsphase konnte erneut mit den osmotischen Wechselschocks begonnen werden.

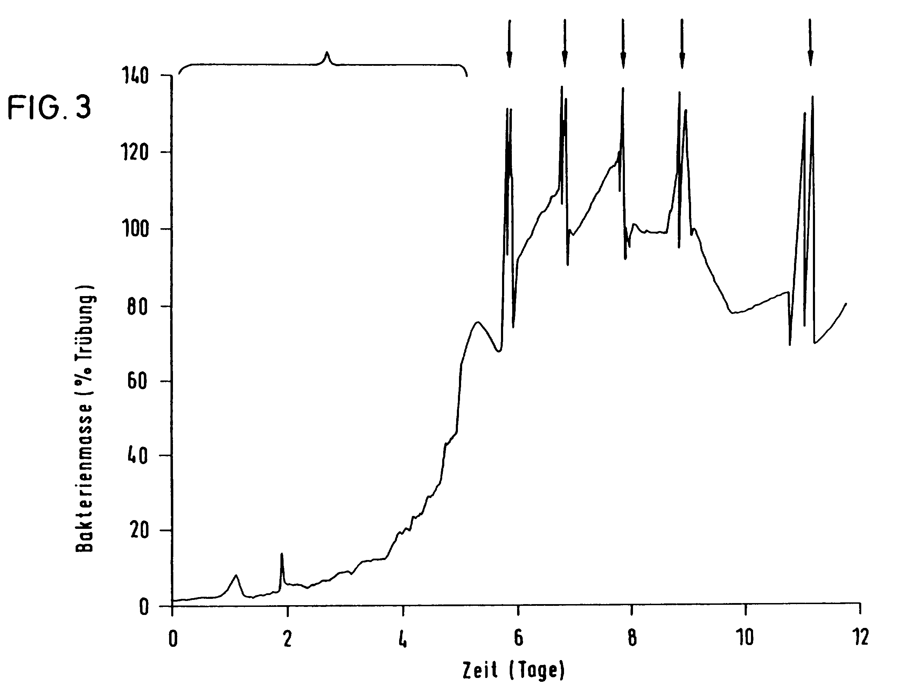

   Figur 3 zeigt einen typischen Verfahrensverlauf, bei dem 5 Wechselschocks durchgeführt worden sind. Die Zeitpunkte der einzelnen Wechselschocks sind durch die in der Figur angegebenen Pfeile gekennzeichnet. In Figur 3 sind auf der Abszisse die Zeit in Tagen und auf der Ordinate die Bakterienmasse in % Trübung angegeben. Es wurden Halomonas elongata Zellen in einer Hochzelldichtefermentation kultiviert.

## Patentansprüche

1. Verfahren zur in vivo Gewinnung von Inhaltsstoffen aus Zellen, ausgenommen Cyanobakterien, wobei die betreffenden Zellen von einem ersten Zustand in einen zweiten Zustand überführt werden (Schock), wodurch die Zellen gezwungen werden, durch Abgabe von Zellinhaltsstoffen in die Umgebung oder Synthese von Zellinhaltsstoffen zu reagieren, um eine Anpassung der Zellen an die Bedingungen des zweiten Zustandes zu erreichen, mit der Maßgabe, daß diese Verfahrensschritte mindestens zweimal (Wechselschock) durchlaufen werden, dadurch gekennzeichnet, daß der Schock durch schnelle Veränderung der osmotischen Verhältnisse in der Umgebung der Zellen hervorgerufen wird, indem die Zellen von einer Osmolarität entsprechend bis zu 30 Osmol in eine Umgebung mit möglichst geringer Osmolarität überführt werden und umgekehrt.

2. Verfahren nach Anspruch 1, wobei zusätzlich weitere Maßnahmen zur Erhöhung der Permeabilität von Zellmembranen, wie Elektroporation, oder Zusatz von permeabilisierenden Substanzen, angewendet werden, oder schnelle Veränderung des in der Umgebung der Zellen herrschenden Druckes, Temperatur, Konzentration von Stoffen, pH-Wert oder Kombinationen dieser Maßnahme vorgenommen werden.

3. Verfahren nach mindestens Anspruch 1 und/oder 2, wobei dafür Sorge getragen wird, daß zwischen den Wechseln quasi-stationäre Bedingungen zwischen Zelle und umgebenden Medium erreicht werden.

4. Verfahren nach mindestens einem der Ansprüche 1 bis 3, wobei die Zellen Mikroorganismen, wie Bakterien, Pilze, Hefen, Algen, Rotalgen oder Zellen höherer Organismen, wie tierische oder pflanzliche Zellkulturen sowie tierische und pflanzliche Gewebe, oder Organe sind.

5. Verfahren nach mindestens einem der Ansprüche 1 bis 4, wobei die Zellen genetisch manipulierte Zellen sind, die bestimmte Stoffe synthetisieren und in Folge des Wechselschocks sezernieren.

6. Verfahren nach Anspruch 1, wobei der Wechsel der Osmotischen Verhältnisse der Umgebung der Zellen durch Austausch des Mediums erfolgt und/oder durch Veränderung der Osmolarität des Mediums.

7. Verfahren nach mindestens einem der Ansprüche 1 bis 6, dadurch gekennzeichnet, daß die Zellen Mikroorganismen wie Proteobakterien, insbesondere Halomonadaceae, oder Firmicutes, insbesondere Marinococci, sind.

8. Verfahren nach einem der Ansprüche 1 bis 7, wobei der Wechsel der Osmolarität von 1 Osmol bis 5 Osmol erfolgt.

9. Verfahren nach mindestens einem der Ansprüche 1 bis 8, wobei die Umgebung der Zellen durch eine Filtration, wie Querstromfiltration, ausgetauscht wird.

10. Verfahren nach mindestens einem der Ansprüche 1 bis 9, wobei die Zellen immobilisiert sind und/oder aggregiert sind.

11. Verfahren nach mindestens einem der Ansprüche 1 bis 10, wobei aus den Zellen die zur Anpassung an den ersten Zustand, beispielsweise hohe Osmolarität, produzierten Stoffe, welche die Zellen im zweiten Zustand abgegeben haben, aus dem Medium isoliert werden.

12. Verfahren nach Anspruch 11, wobei Stoffe zur Osmoregulation und/oder Osmotika, wie Ectoin und/oder Derivate des Ectoins isoliert werden.

## Claims

1. A method for the in vivo recovery of substances contained in cells, with the exception of cyanobacteria, wherein said cells are transferred from a first state to a second state (shock) whereby the cells are forced to respond by releasing substances contained in the cells into the environment or by synthesizing substances contained in cells in order to achieve an adaption of the cells to the conditions of the second state, with the proviso that these process steps are run at least twice (alternating shock), characterized in that said shock is brought about by a rapid change of the osmotic conditions in the environment of the cells by transferring the cells from an osmolarity corresponding to up to 30 osmol to an environment with as low as possible an osmolarity, and vice versa.

2. The method according to claim 1, wherein further measures for increasing the permeability of cell membranes, such as electroporation, or addition of permeabilizing substances, are additionally employed, or a rapid change of the pressure prevailing in the environment of the cells, temperature, concentration of materials, pH value, or combinations of such measures are performed.

3. The method according to at least claim 1 and/or 2, wherein it is ensured that quasistationary conditions between the cell and the surrounding medium are achieved between the alternations.

4. The method according to at least one of claims 1 to 3, wherein said cells are microorganisms, such as bacteria, fungi, yeasts, algae, red algae, or cells of higher organisms, such as animal or vegetable cell cultures, or animal and vegetable tissues or organs.

5. The method according to at least one of claims 1 to 4, wherein said cells are genetically engineered cells which synthesize certain substances and secrete them as a consequence of the alternating shock.

6. The method according to claim 1, wherein the alternation of the osmotic conditions in the cells' environment is effected by changing the medium and/or by changing the osmolarity of the medium.

7. The method according to at least one of claims 1 to 6, characterized in that said cells are microorganisms such as proteobacteria, especially Halomonadaceae, or Firmicutes, especially Marinococci.

8. The method according to any of claims 1 to 7, wherein said alternation in osmolarity is from 1 osmol to 5 osmol.

9. The method according to at least one of claims 1 to 8, wherein the cells' environment is changed by filtration, such as transverse flow filtration.

10. The method according to at least one of claims 1 to 9, wherein said cells are immobilized and/or aggregated.

11. The method according to at least one of claims 1 to 10, wherein the substances produced by the cells for adaption to the first state, for example, high osmolarity, and released by the cells in the second state are isolated from the medium.

12. The method according to claim 11, wherein substances for osmoregulation and/or osmotic agents, such as ectoine and/or derivatives of ectoine, are isolated.

## Revendications

1. Procédé d'obtention in vivo de constituants cellulaires, à l'exception des cyanobactéries, dans lequel les cellules considérées sont converties d'un premier état à un deuxième état (choc) ce en conséquence de quoi les cellules sont obligées de réagir, en cédant des constituants cellulaires à l'environnement, ou en synthétisant des constituants cellulaires, pour arriver à une adaptation des cellules aux conditions du deuxième état, à la condition que ces étapes du procédé soient parcourues au moins deux fois (choc alterné), caractérisé en ce que le choc est provoqué par une variation rapide des conditions osmotiques dans le milieu ambiant des cellules, les cellules dont l'osmolarité va jusqu'à 30 osm étant transférées dans un milieu ambiant dont l'osmolarité est aussi petite que possible, et inversement.

2. Procédé selon la revendication 1, dans lequel on fait appel en outre à des mesures supplémentaires pour augmenter la perméabilité des membranes cellulaires, notamment à une électroporation, ou encore à l'addition de substances à effet perméabilisant, ou encore on procède à une variation rapide de la pression, de la température, de la concentration de substances, du pH, régnant dans le milieu ambiant des cellules, ou à une combinaison de ces mesures.

3. Procédé selon au moins l'une des revendications 1 et/ou 2, dans lequel on veille à arriver entre les alternances à des conditions quasi-stationnaires entre la cellule et le milieu ambiant.

4. Procédé selon au moins l'une des revendications 1 à 3, dans lequel les cellules sont des micro-organismes tels que des bactéries, des champignons, des levures, des algues, des algues rouges ou des cellules d'organismes supérieurs tels que des cultures cellulaires animales ou végétales, ou encore des tissus animaux ou végétaux, ou des organes.

5. Procédé selon au moins l'une des revendications 1 à 4, dans lequel les cellules sont des cellules ayant subi une manipulation génétique, qui synthétisent certaines substances, et les sécrètent en conséquence du choc alterné.

6. Procédé selon la revendication 1, dans lequel le changement des conditions osmotiques du milieu ambiant des cellules est réalisé par remplacement du milieu et/ou par une modification de l'osmolarité du milieu.

7. Procédé selon au moins l'une des revendications 1 à 6, caractérisé en ce que les cellules sont des micro-organismes tels que des protéobactéries, en particulier Halomonadaceae, ou encore Firmicutes, en particulier Marinococci.

8. Procédé selon l'une des revendications 1 à 7, dans lequel la variation d'osmolarité a lieu de 1 à 5 osm.

9. Procédé selon au moins l'une des revendications 1 à 8, dans lequel le milieu ambiant des cellules est remplacé grâce à une filtration, par exemple une filtration à flux transversal.

10. Procédé selon au moins l'une des revendications 1 à 9, dans lequel les cellules sont immobilisées et/ou agrégées.

11. Procédé selon au moins l'une des revendications 1 à 10, dans lequel, les substances provenant des cellules, produites pour permettre une adaptation au premier état, par exemple une osmolarité élevée, et que les cellules ont cédé dans le deuxième état, sont isolées du milieu.

12. Procédé selon la revendication 11, dans lequel on isole des substances pour l'osmorégulation et/ou des substances osmotiques telles que l'ectoïne et/ou des dérivés de l'ectoïne.
